# EUROPEAN PATENT SPECIFICATION

(11) **EP 3 133 818 B1**
(45) Date of publication and mention of the grant of the patent: **17.10.2018**
(21) Application number: 16184958.3
(22) Date of filing: 19.08.2016
(51) Int. Cl.: H04N 21/258, H04N 21/422, H04N 21/442, H04N 21/466

(54) **METHOD AND APPARATUS FOR CONTROLLING DEVICE, AND SMART MAT**
VERFAHREN UND VORRICHTUNG ZUR STEUERUNG EINER VORRICHTUNG UND INTELLIGENTE MATTE
PROCÉDÉ ET APPAREIL PERMETTANT DE COMMANDER UN DISPOSITIF ET TAPIS INTELLIGENT

(30) Priority: 20.08.2015 CN 201510515888
(43) Date of publication of application: 22.02.2017
(73) Proprietor: Xiaomi Inc., Beijing 100085 (CN)
(72) Inventor: WU, Ke, BEIJING, 100085 (CN); LIU, Xinyu, BEIJING, 100085 (CN)
(74) Representative: Delumeau, François Guy

(56) References cited:
- WO-A1-2014/151577
- US-A1- 2008 230 497
- US-A1- 2009 091 529
- US-A1- 2010 045 609
- US-A1- 2012 124 456
- US-A1- 2015 039 259
- Thiago Teixeira: "A Survey of Human-Sensing: Methods for Detecting Presence, Count, Location, Track, and Identity", , 1 September 2010 (2010-09-01), XP055101195, Retrieved from the Internet: URL:http://www.cse.buffalo.edu/~wenyaoxu/c ourses/cse741/papers/human_sensing.pdf [retrieved on 2014-02-10]
- ANDREAS RIENER ET AL: "Supporting Implicit Human-to-Vehicle Interaction: Driver Identification from Sitting Postures", PROCEEDINGS OF THE FIRST ANNUAL INTERNATIONAL SYMPOSIUM ON VEHICULAR COMPUTING SYSTEMS, 22 July 2008 (2008-07-22), XP055351946, Gent, BELGIUM DOI: 10.4108/ICST.ISVCS2008.3545
- MIDDLETON L ET AL: "A Floor Sensor System for Gait Recognition", AUTOMATIC IDENTIFICATION ADVANCED TECHNOLOGIES, 2005. FOURTH IEEE WORK SHOP ON BUFFALO, NY, USA 17-18 OCT. 2005, PISCATAWAY, NJ, USA,IEEE, 17 October 2005 (2005-10-17), pages 171-176, XP010856517, ISBN: 978-0-7695-2475-7

## Description

### TECHNICAL FIELD

The embodiments of the present disclosure relate to the technical field of smart homes, particularly to a method and apparatus for controlling a device, and an smart mat.

### BACKGROUND

Generally, a user is used to opening a device to view and select information that the user prefers when lying or sitting on a mat in daily life, wherein the mat may be a mattress, a seat cushion, a sofa cushion and so on. Taking a mattress for example, the user usually gets used to opening a device to play contents such as a television show, a movie, an electronic book and so on that the user prefers when lying on a mattress. In other words, nowadays, a mat is a place where content playing frequently happens. However, in the related art, there is rarely a solution which addresses improving the content playing in such a place.

The document US2008230497A1 discloses an edge display system, which includes at least one shelf defining a surface and at least one electronic display device.

The document "A Survey of Human-Sensing :Methods for Detecting Presence, Count, Location, Track, and Identity" summarizes the existing solutions from various disciplines, to guide the creation of new system and point toward future research directions.

The document "Supporting Implicit Human-to-Vehicle Interaction: Driver Identification from Sitting Postures" discloses a driver identification system for vehicular services.

The embodiments of the present disclosure are presented to solve the problem of the lack of solution enabling improving the content playing which frequently happens on a mat in the related art.

### SUMMARY

Accordingly, the present disclosure provides a method and an apparatus for controlling a device, in accordance with claims which follow,

A method for controlling a device implemented by a terminal is provided according to Claim 1.

In this way, the recommended content for the user who is situated on the smart mat is automatically played. Therefore the content playing which happens on the mat is improved specially for the user of the mat, thus improving playing efficiency and bringing more convenience to the user.

According to the invention, the step that the identify information of the user on the smart mat is determined includes that:
whether an object on the smart mat is a user is detected; and
if a user is detected, identity information of the user is determined.

In this way, other objects which are not human bodies are filtered, only a user is identified to determine identity information thereof while other objects are not identified, thus improving the efficiency in determining identity information.

Alternatively, the step that whether the object on the smart mat is a user is detected may include that:
temperature data of the object on the smart mat is acquired; and
if the temperature data is within a pre-stored temperature range of human body, it is determined that the object on the smart mat is a user.

In this way, the judgment efficiency is improved.

Alternatively, the step that whether the object on the smart mat is a user is detected may include that:
pressure information of the object on the smart mat is acquired;
a shape of the object on the smart mat is determined according to points where the pressure information is acquired on the smart mat;
the determined shape is compared with a pre-stored shape of a human body to obtain a first similarity; and
when the first similarity is larger than a first preset threshold, it is determined that the object is a user.

In this way, the judgment efficiency is improved.

Alternatively, the step that whether the object on the smart mat is a user is detected may include that:
pressure information of the object on the smart mat is acquired;
a pressure area is determined according to points where the pressure information is acquired on the smart mat;
according to a ratio of the pressure information to the pressure area, density of the object on the smart mat is acquired; and
if the density is within a pre-stored density range of human body, it is determined that the object is a user.

In this way, whether the object on the smart mat is a user is determined according to the density, thus improving the judgment accuracy.

According to the invention, the step that the identity information of the user is determined includes that:
difference values between physiological parameters and sample physiological parameters in a data list are determined, wherein the physiological parameters include the pressure information, the pressure area or the density and the data list records corresponding relations between sample physiological parameters and identity information of users;
a target difference value within an allowable fluctuation range is selected from the difference values, and a target sample physiological parameter corresponding to the target difference value is determined; and
the identity information of the user corresponding to the target sample physiological parameter is acquired from the data list, wherein the data list records correspondence relationships between sample physiological parameters and identity information of users.

Since the physiological parameters have been determined when the user is determined, it is unnecessary to acquire other data when the identity information is determined, thus saving resources.

According to the invention, a manner for creating the data list includes that:
history physiological parameters of a user in a first history period are acquired from a database;
history physiological parameters of the user are averaged to obtain sample physiological parameters of the user; and
correspondence relationships between identity information of the user and sample physiological parameters are established so as to create the data list.

In this way, the reliability of the sample physiological parameters is improved.

According to the invention, a manner for determining the allowable fluctuation range includes that:
an exercise parameter of the user within a second history period is acquired;
the exercise parameter is compared with an upper exercise limit and a lower exercise limit of the user; and
the allowable fluctuation range is determined according to a comparison result of the comparison.

The allowable fluctuation range is adjusted according to the exercise parameter, which considers a fact that the physiological parameters change with the exerciseof a user, thereby improving the accuracy of the allowable fluctuation range and further improving the accuracy in determining the identity information.

Alternatively, the step that the recommended content for the user is determined according to the identity information of the user may include that:
history playing information of the user during a third history period is read according to the identity information of the user; and
the history playing information is analyzed to acquire the recommended content for the user.

In this way, the accuracy in acquiring the recommended content is improved.

Alternatively, the step that the recommended content for the user is determined according to the identity information of the user may include that:
when there are at least two users on the smart mat, a sequence that the at least two users contact the smart mat is acquired;
recommended content corresponding to each of the at least two users is determined according to the identity information of the at least two users; and
the recommended content of the at least two users is sorted according to the sequence to acquire a recommended content list.

Since a sequence of recommended content is determined according to a sequence that users contact the smart mat, a user who contacts the mat first has the priority to view his/her recommended content, thus improving user experience.

Alternatively, the step that the instruction for playing the recommended content is sent to the device associated with the smart mat may include that:
a wireless connection with the device associated with the smart mat is created ; and
a booting instruction and the instruction for playing the recommended content are sent to the device through the wireless connection.

In this way, automatic booting can be implemented, recommended content can be played and user experience are improved.

A terminal for controlling a device is provided according to Claim 8

Alternatively, the object judging sub-unit may include:
a temperature data acquiring module, configured to acquire temperature data of the object on the smart mat; and
a first judging module configured to determine that the object on the smart mat is a user when the temperature data is in a pre-stored temperature range of human body.

Alternatively, the object judging sub-unit may include:
a first pressure information acquiring module, configured to acquire pressure information on the smart mat;
a shape determining module, configured to determine a shape of the object on the smart mat according to points where the pressure information is capable of being acquired on the smart mat;
a first similarity determining module, configured to compare the determined shape with a pre-stored shape of a human body to obtain a first similarity; and
a second judging module, configured to determine that the object is a user when the first similarity is larger than a first preset threshold.

Alternatively, the object judging sub-unit may include:
a second pressure information acquiring module, configured to acquire pressure information on the smart mat;
a pressure area determining module, configured to determine a pressure area according to points where the pressure information is capable of being acquired on the smart mat;
a density determining module, configured to according to a ratio of the pressure information to the pressure area, acquire density of the object on the smart mat; and
a third judging module, configured to determine that the object is a user when the density is in a pre-stored density range of human body.

According to the iinvention, the information determining sub-unit includes:
a difference value calculating module, configured to calculate difference values between physiological parameters and sample physiological parameters in a data list, wherein the physiological parameters include the pressure information, the pressure area or the density;
a target sample physiological parameter determining module, configured to select a target difference value in an allowable fluctuation range from the difference values, and determine a target sample physiological parameter corresponding to the target difference value; and
a first identity information determining module, configured to acquire identity information of a user corresponding to the target sample physiological parameter from the data list, wherein the data list records corresponding relations between sample physiological parameters and identity information of users.

According to the iinvention, the information determining sub-unit further includes:
a history physiological parameter acquiring module, configured to acquire history physiological parameters of each user in a first history period from a database;
a sample physiological parameter determining module, configured to average the history physiological parameters of the each user to obtain sample physiological parameters of the each user; and
a data list determining module, configured to create corresponding relations between identity information of users and sample physiological parameters and obtain the data list.

According to the iinvention, the information determining sub-unit further includes:
an exercise parameter acquiring module, configured to acquire an exercise parameter of the user within a second history period;
a comparing module, configured to compare the exercise parameter with an upper exercise limit and a lower exercise limit of the user; and
a fluctuation range determining unit, configured to determine the allowable fluctuation range of the exercise parameter of the user according to a comparison result.

Alternatively, the information determining sub-unit may include:
a biologic characteristic information acquiring module, configured to acquire biologic characteristic information of the user, wherein the biologic characteristic information includes fingerprint information, palm print information and face information;
a comparing unit, configured to compare the acquired biologic characteristic information with pre-stored biologic characteristic information of at least one valid user; and
a second identity information determining module configured to read, when a second similarity of the acquired biologic characteristic information to biologic characteristic information of one valid user is larger than a second preset threshold, pre-stored identity information of the one valid user.

Alternatively, the information determining sub-unit may include:
an account information reading module, configured to read account information used by the user to log in an App on a terminal; and
a third identity information determining module, configured to determine the identity information of the user according to the account information.

Alternatively, the recommended content determining unit may include:
a history watching information acquiring sub-unit, configured to read history watching information of the user in a third history period according to the identity information of the user; and
a first recommended content determining sub-unit, configured to analyze the history watching information to acquire the recommended content of the user.

Alternatively, the recommended content determining unit may include:
a sequence obtaining sub-unit configured to acquire, when there are at least two users on the smart mat, a sequence that the at least two users contact the smart mat;
a second recommended content determining sub-unit, configured to determine recommended content corresponding to each of the at least two users according to the identity information of the at least two users; and
a recommended content list determining sub-unit, configured to sort the recommended content of the at least two users according to the sequence to acquire a recommended content list.

Alternatively, the instruction sending unit may include:
a connection creating sub-unit, configured to create a wireless connection with the device associated with the smart mat; and
an instruction sending sub-unit, configured to send a booting instruction and the instruction for playing the recommended content to the device through the wireless connection.

An apparatus is provided according to a third aspect of the embodiments of the present disclosure. The apparatus includes:
a processor; and
a memory configured to store an instruction executable by the processor,
wherein the processor is configured to implement the method of Claim 1.

The advantages and features of the apparatus and the smart mat according to the embodiments of the present disclosure are the same with those of the above described method and will not be repeated here.

Alternatively, the steps of the method for controlling a device are determined by computer program instructions.

Consequently, according to a fifth aspect, the invention is also directed to a computer program for executing the steps of the method for controlling a device as described above when this program is executed by a computer.

This program can use any programming language and take the form of source code, object code or a code intermediate between source code and object code, such as a partially compiled form, or any other desirable form.

The invention is also directed to a computer-readable information medium containing instructions of a computer program as described above.

The information medium can be any entity or device capable of storing the program. For example, the support can include storage means such as a ROM, for example a CD ROM or a microelectronic circuit ROM, or magnetic storage means, for example a diskette (floppy disk) or a hard disk.

Alternatively, the information medium can be an integrated circuit in which the program is incorporated, the circuit being adapted to execute the method in question or to be used in its execution. It is to be understood that the general description above and the detail description hereinafter are only exemplary and explanatory, but can not limited the present disclosure.

### BRIEF DESCRIPTION OF THE DRAWINGS

The accompanying drawings herein, which are incorporated into the specification and constitute a part of the specification, illustrate embodiments consistent with the present disclosure, and serve to explain the principles of the embodiments of the present disclosure together with the specification.
Fig. 1A is a flowchart of a method for controlling a device according to an exemplary embodiment of the present disclosure;
Fig. 1B is a schematic diagram of playing recommended content according to an exemplary embodiment of the present disclosure;
Fig. 2 is a schematic diagram of an application scenario of a method for controlling a device according to an exemplary embodiment of the present disclosure;
Fig. 3 to Fig. 15 are block diagrams of an apparatus for controlling a device according to an exemplary embodiment of the present disclosure; and
Fig. 16 is a structural diagram of a control apparatus applied to a device according to an exemplary embodiment of the present disclosure.

### DETAILED DESCRIPTION

The exemplary embodiments will be described in details herein, examples of which are illustrated in the accompanying drawings. Unless otherwise expressed, the same numbers in different accompanying drawings represent the same or similar elements when the accompanying drawings are described hereinafter. The implementation modes described in the following exemplary embodiments do not represent all implementation modes consistent with the present disclosure. On the contrary, they are merely examples of apparatuses and methods as described in details in the appended claims and consistent with some aspects of the present disclosure.

Terms used in the present disclosure are only intended to describe specific embodiments, but are not intended to limit the present disclosure. Singular forms "a", "said" and "the" used in the present disclosure and the appended claims also aim to include plural forms except that the context clearly indicates other meanings. It it also to be understood that, the term "and/or" used herein indicates and includes any or all possible combinations of one or more listed items that are associated.

It is to be understood that although the embodiments of the present disclosure may apply terms including first, second, third and so on to describe various information, these information should not be limited to these terms. These terms are only used for distinguishing information of the same type from each other. For example, first information may be also called second information without departing from the scope of the embodiments of the present disclosure, and similarly, second information may be also called first information, which depends on context. For example, the word "if" used here may be interpreted as "at the moment when......" or "when......" or "in response to confirmation".

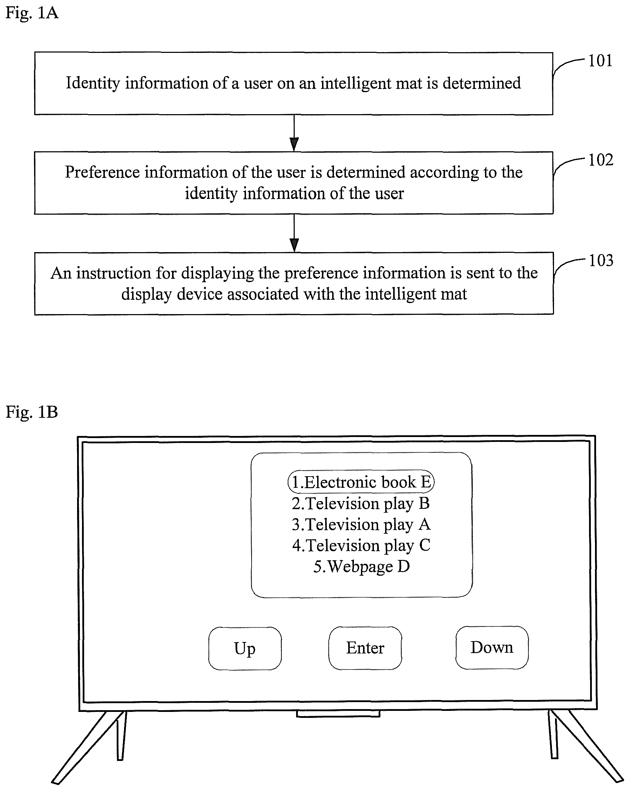

As shown in Fig. 1A, Fig. 1A is a flowchart of a method for controlling a device according to an exemplary embodiment. The method may be implemented by a terminal, and may be also applied to an smart mat. Herein, a smart mat means a mat which can communicate with a terminal and can implement data transmission and control. The present embodiment illustrates an implementation of the method by a terminal as an example. The method may include the following steps.

Step 101: Identity information of a user situated on an smart mat is determined.

Beofre this step 101, an optional step of determining whether an object situated on a smart mat is a user can be carried out. This step will be explained hereinafter.

The terminal in the present disclosure may be any smart terminal having an Internet access function. For example, the terminal may be a smart phone, a tablet computer, a Personal Digital Assistant (PDA) and so on, wherein the terminal may access a router by a wireless local area network, and access a server in a public network through the router. An Application (App) is installed on the terminal. A device (e.g. a personal computer or a mobile phone) may be associated with (e.g, via Wireless Fidelity or a mobile network) the smart mat and may be controlled by the App on the terminal. A network communication module capable of communicating with the terminal is provided in the smart mat and the device. The smart mat in the embodiments of the present disclosure may be realized in the form of a mattress, a seat cushion, a sofa cushion and so on, and the embodiments of the present disclosure mainly illustrate a mattress as an example.

The optional step mentionned above aims to determine that an object on the smart mat is a user, but not an animal or another object, such as a pillow, a quilt and so on. For example, when a user lies on a mattress or sits on a sofa cushion, it may be first detected whether the object on the smart mat is a user, and when a user is detected, identity information of the user is determined. It should be noted that the step of detecting whether the object on the smart mat is a user is optional but not necessary.

Several temperature sensors may be arranged on the smart mat in the embodiments of the present disclosure, and several pressure sensors may be also arranged thereon. The temperature sensors and the pressure sensors may be arranged uniformly in a mesh pattern on the smart mat. The temperature sensors are configured to detect the temperature of the object on the smart mat, and the pressure sensors are configured to detect a received pressure. Each pressure sensor corresponds to a detection point.

In the step of the embodiments of the present disclosure, whether the object on the smart mat is a user may be detected by one or more of the following manners.

### Manner 1

The terminal uses pressure sensors arranged on the smart mat to acquire pressure information on the smart mat, and determines the shape of the object on the smart mat according to points where the pressure information is acquired on the smart mat. In an embodiment of the present disclosure, the shape of the object may be determined based on outermost pressure sensors among the pressure sensors which acquire the pressure information. The determined shape is compared with a pre-stored shape of human body, so as to obtain a first similarity, and if the first similarity is larger than a first preset threshold, it is determined that the object is a user, and otherwise, it is determined that the object is not a user. The pre-stored shape of human body here may be a shape of a whole human body, or may be a shape of a part of a human body. For mats such as a seat cushion, an determined shape is usually a shape of a part of a human body, for example, the buttocks and legs of the user sitting on the seat cushion.

In the manner of the pressure information on the smart mat present disclosure, the shape of the object on the smart mat is determined according to the pressure information, and whether the object is a user is judged according to the shape This solution is simple to realize and thus improves the judgment efficiency.

### Manner 2

The terminal uses pressure sensors to acquire pressure information of the object on the smart mat and determines a pressure area (i.e. the area composed by pressure sensors which receive pressure information) according to points where the pressure information is acquired on the smart mat, then calculates the ratio of the pressure information to the pressure area so as to obtain a density of the object on the smart mat, then compares the density of the objet with a pre-stored density range of human body, and if the calculated density is in the pre-stored density range of human body, then determines that the object is a user, and otherwise, determines that the object is not a user.

In the manner of the embodiments of the present disclosure, whether the object on the smart mat is a user is judged according to density. Since the density of a human body is significantly different from the density of other objects, the judgment accuracy can be improved.

Additionally, the pre-stored density range of human body may be determined according to history data. For example, history data comprises one or more set of pressure information and pressure area(s) on the mattress where users lie on his/her back.A density range of a human body lying on his/her back is thus acquired according to a ratio or an average of ratios of the pressure information to the pressure area(s) obtained from the history data. History data may also comprises one or more set of pressure information and pressure area(s) of a mattress on which users sit, so as to acquire a density range of a sitting human body.

### Manner 3

The terminal uses temperature sensors to acquire temperature data of the object on the smart mat, and compares the acquired temperature data with a pre-stored temperature range of human body. If the temperature data is in the pre-stored temperature range of human body, the terminal determines that the object on the smart mat is a user, and otherwise, determines that the object on the smart mat is not a human body. Herein, since various parts of a human body have roughly the same temperature, temperature data acquired by some temperature sensors only may be compared with the temperature range of human body. Some temperature data may be respectively compared with the temperature range of human body. The temperature data of the object may be also calculated to be an average of the temperature data acquired by the temprature sensors, which is compared with the prestored temperature range of human body. Alternativly, the temperature data of the object is calculated based on a certain algorithm known in the art to reflect an overall temperature of the object, the calculated temprature data being compared with the prestored temperature range of human body.

In the method according to this embodiment, whether the object is a user is judged according to temperature. This solution is also simple to implement, thereby improving the judgment efficiency.

It is to be understood that manners for detecting whether an object on the smart mat is a user are not limited to the manners described above, which may be manners for determining a user in the related art. Besides, whether the object is a user may be judged by only one manner so as to improve the judgment efficiency, or may be judged by combining a plurality of manners, so as to improve the judgment accuracy. For example, the judgment is performed by combining the first manner and the second manner, or by combining the first manner and the third manner, and so on.

Subsequently, identity information of the user situated on the smart mat may be determined.

A plurality of users may use the smart mat in a family, and the users who have used the smart mat may be called valid users. Identity information of a valid user may be pre-stored in the terminal, for example information identifying the user such as a name, a username, an account, a body height, a body weight, face image information and a mobile phone number and so on. The identity information of the user may be determined by the following modes.

Mode 1: The identity information of the user is determined according to physiological parameters.

First, difference values between physiological parameters and sample physiological parameters in a data list are calculated.

Herein, the physiological parameters are those calculated in real-time for the user who is currently using the smart mat while the sample physiological parameters are those recorded in the data list for valid users. The difference value between a physiological parameter and a sample physiological parameter is for example a difference of pressure, pressure area, density, height of a user, etc.The physiological parameters may include pressure information, pressure area, the density of a user, the height of the user and so on. When the physiological parameters are information (such as pressure information, pressure area, and the density of a user) acquired during a previous step of determining whether the objet is a user, the physiological parameters may be acquired directly., The physiological parameters may be otherwise acquired. For example, in the case of a smart mattress, the terminal may determine the body length (body height) of a user lying on the smart mattress based on points (e.g. pressure sensors) between which the distance is the longest along the length of the smart mattress. A user usually lies along the length of the smart mat. Generally, points between which the distance is the longest along the length of the smart mat correspond to the head and the feet of the user. An approximate height of the user may be determined by this mode. A data list records correspondance relationships between sample physiological parameters and identity information of users. Different sample physiological parameters correspond to different valid users, thus the identity of a corresponding user may be determined according to sample physiological parameters.

Secondly, each of the difference values is compared with an allowable fluctuation range. A target difference value that falls in the allowable fluctuation range is selected from the difference values, and a target sample physiological parameter corresponding to the target difference value is determined. The identity information of a user corresponding to the target sample physiological parameter is acquired from the data list. A physiological parameter of a user may change as time goes by, a body weight may change, for example, thereby resulting in a change in pressure information and a pressure area; therefore, physiological parameters corresponding to difference values within the allowable fluctuation range may belong to the same user.

It may be understood that the identity information of the user may be determined by only one physiological parameter. Alternatively, the identity information of the user may be determined by a plurality of physiological parameters so as to improve the accuracy in the determining of the identity information. For example, the identity information of the user is determined by both the pressure information and the pressure area. If a difference value between pressure information and sample pressure information falls in an allowable fluctuation range, and if a difference value between a pressure area and a sample pressure area is in an allowable fluctuation range, the corresponding identity information is determined.

For example, pressure information of a user situated on the smart mat is acquired, a pressure area is determined based on points where the pressure information is acquired on the smart mat. A pressure difference value between the acquired pressure information and sample pressure information recorded in the data list is calculated. An pressure area difference value between the acquired pressure area and a sample pressure area recorded in the data list is calculated, wherein the data list records correspondance relationships regarding sample pressure information, sample pressure areas and identity information of users. A group of target sample pressure information and target sample pressure areas is selected, the group having a pressure difference value and a pressure area difference value within allowable fluctuation ranges. Identity information of the user is determined according to the target sample pressure information and the target sample pressure area recorded in the data list.

Additionally, a manner for creating the data list may include the following steps.

Firstly, history physiological parameters of a user during a first history period are acquired from a database. The first history period is a preset recent period of time. For example, the first history period may be the last one week, or the last one month, which may be specifically set as required. Current physical conditions of the user may be truly reflected by history physiological parameters within a recent period of time, thereby improving the reliability of the sample physiological parameters in the data list.

Secondly, history physiological parameters of a user are averaged to obtain a sample physiological parameter of the user. A user has a plurality of history physiological parameters. Physical conditions of a user may be reflected comprehensively by an average of the plurality of physiological parameters of the user, thereby improving the reliability of the sample physiological parameters in the data list.

Finally, correspondance relationships between identity information of users and sample physiological parameters are established and the data list is created.

The identity information of the user may be determined according to sample physiological parameters by checking the established correspondance relationships.

Additionally, the allowable fluctuation range may be also determined by the following way.

Firstly, an exercise parameter (e.g. a running distance, steps, and the number of stairs that a user climb) of a user within a second history period is acquired. The second history period is a preset recent period of time. For example, the second history period may be the last one week, or the last one month, which may be specifically set as required. The second history period may be the same as the first period, or may be different. In the present step, an exercise parameter of the user may be acquired by a portable device associated with the terminal. For example, an exercise parameter of the user in the last one week may be acquired through a smart wrist band.

Secondly, the exercise parameter is compared with an upper exercise limit and a lower exercise limit of the user, wherein the upper exercise limit means a preset upper threshold value, and the lower exercise limit means a preset lower threshold value.

Finally, the allowable fluctuation range of the exercise parameter of the user is determined according to a comparison result of the above comparison.

The upper exercise limit and the lower exercise limit of the user may be preset. The upper exercise limit and the lower exercise limit may be used for measuring a possible changing trend of physiological parameters of the user. For example, when the exercise parameter is larger than the upper exercise limit, it is considered that there is a large amount of exercise, which may result in a decrease in the pressure information and the pressure area (e.g. the user has lost weight), and the allowable fluctuation range may be adjusted accordingly. When the exercise parameter is smaller than the lower exercise limit, it is considered that there is a small amount of exercise, which may result in an increase in the pressure information and the stressed area (e.g. the user has gained weight), and the allowable fluctuation range may be adjusted accordingly. Thus, the allowable fluctuation range may be determined according to a comparison result of comparing the exercise parameter of the user with the upper exercise limit and the lower exercise limit. Alternatively, a specific value of the fluctuation range may be designated.

For example provided that a default allowable fluctuation range is (-10%*w*₀,+10%*w*₀), wherein *w*₀ is a sample pressure information of the user. The allowable fluctuation range may be increased if a user has an extremely small exercise amount recently according to an exercise amount recorded by a wrist band of the user in the last one week. For example, the fluctuation range of pressure information (W) is adjusted to (-10%*w*₀,+20%*w*₀). The allowable fluctuation range may be adjusted to (-20%*w*₀,+10%*w*₀), for example, if the user has an extremely large exercise amount recently.

This solution adjusts the allowable fluctuation range according to the exercise parameter, which takes into account the fact that a physiological parameter changes with the exercise activities of the user, thereby improving the accuracy in determining the identity information.

In another alternative implementation mode, a fluctuation range may be preset. The fluctuation range may be a proportion, and may be also a specific value. For example, a fluctuation range of the pressure information may be set as (-10%*w*₀,+10%*w*₀), wherein *w*₀ is a sample pressure information of the user. For another example, a fluctuation range of the pressure information is set as (-5*kg*,+5*kg*)*.*

Mode 2: The identity information of the user is determined according to biologic characteristic information.

Firstly, biologic characteristic information of the user is acquired. The biologic characteristic information may include for example fingerprint information, palm print information, face information and so on. For example, the terminal may recognize the face of the user by a built-in camera, or may recognize the fingerprint of the user by a built-in fingerprint recognition module.

Secondly, the acquired biologic characteristic information is compared with pre-stored biologic characteristic information of a valid user.

Biologic characteristic information may change as time goes. Accordingly, the acquired biologic characteristic information may be directly compared with pre-stored biologic characteristic information of a valid user.

Finally, when a second similarity of the acquired biologic characteristic information to pre-stored biologic characteristic information of a valid user is larger than a second preset threshold, the pre-stored identity information of the valid user is read.

When the acquired biologic characteristic information is similar to biologic characteristic information of a valid user, it may be determined that the object on the smart mat is the valid user, thus the pre-stored identity information is determined to be the identity information of the object.

In an embodiment of the present disclosure, biologic characteristic information and the corresponding identity information of a valid user are pre-stored in the terminal. After a user currently situated on the smart mat is determined according to biologic characteristic information, corresponding identity information may be read from a database.

Mode 3: The identity information of the user is determined according to account information used by the user to log in an App on the terminal.

Account information used by the user to log in an App on the terminal is read. Before using the smart mat, the user may log in any App on the terminal, and account information for logging in the App may be acquired to determine the identity information of the user according to the account information. In an embodiment, the account information may be directly determined as the identity information. In other words, the identity information is in the form of the account information. Alternatively, identity information may be determined according to a pre-created relationship between account information and identity information of a user. For example, the account information is an account number, and the identity information is a name. A relationship between the account number and the name is created in advance. After the account number is acquired, the corresponding name may be determined according to the account number.

Step 102: Recommended content for the user is determined according to the identity information of the user.

Herein, the recommended content is data to be played to the user. The recommended content may include an audio, a video, an image, a webpage, an electronic book and so on.

In an alternative implementation mode, history watching information of the user in a third history period may be obtained according to the identity information of the user. The history watching information is analyzed to acquire the recommended content for the user, wherein the third history period is a preset recent period of time. For example, the third history period may be the last one week, or the last one month, which may be specifically set as required. The third history period may be the same as the first history period or the second history period, or may be different from the first history period or the second history period.

A preset strategy may be applied in the analyzing of the history watching information to determine the recommended content. For example, information that is not completely played to the user has the highest priority to play. For example, the user shuts down without finishing playing a movie "XXX" when watching the movie last time, then the playing progress of the movie may be acquired this time to continue playing the movie "XXX". Alternatively, information may be prioritized to play according to playing frequencies of the information. Thus information are determined to be sequencely played according to their priorities.. For example, it is analyzed according to a statistics of the history watching information that the user prefers to watch American television series. The recommended content is thus determined to be American television series. For another example, it is analyzed according to a statistics of the history watching information that, within one week, the user watched a television show A for 5 times, watched a television show B for 6 times, watched a television show C for 3 times, browsed a webpage D twice, and read an electronic book E for 7 times, then the recommended content is determined as follows: the electronic book E, the television play B, the television play A, the television play C and the webpage D.

In another alternative implementation mode, when there are at least two users on the smart mat, a sequence that the at least two users contact the smart mat is acquired; recommended content corresponding to each of the at least two users is determined according to identity information of the at least two users; the recommended content is sorted according to the sequence to acquire a recommended content list. The present embodiment illustrates the situation where there is a plurality of users on the smart mat. Each user corresponds to recommended content, the users may be sorted according to a sequence of contact with the smart mat, and recommended content corresponding to the users is accordingly sorted, thus recommended content of a user who contacts the smart mat first will be played first.

Step 103: An instruction for playing the recommended content is sent to a device associated with the smart mat.

The present step may send an instruction for playing the recommended content to a device associated with the smart mat so that the device can play the recommended content. The device may be a television, a large screen display, a projector and so on. The device may locally acquire, according to the instruction, data corresponding to the recommended content, may also acquire data corresponding to the recommended content from a cloud, and may also acquire data corresponding to the recommended content from a terminal. For example, the device may acquire data corresponding to the recommended content from a terminal, including a mobile phone, a tablet computer, a laptop computer and so on of the user.

In an alternative implementation mode, the instruction for playing the recommended content may be an instruction for directly playing data corresponding to the recommended content. For example, when there is a plurality of pieces of recommended content, specific data corresponding to the recommended content may be played in turn. For example, American television series A may be played first according to the recommended content, American television series B are played when a switching instruction is received or a preset moment arrives, and so on.

In another alternative implementation mode, the instruction for playing the recommended content may be an instruction for playing the recommended content list. For example, Fig. 1B shows a schematic diagram of playing recommended content according to an exemplary embodiment of the present disclosure. A prompt box pops up on the device, and a recommended content list "electronic book E, television play B, television play A, television play C and webpage D" is played in the prompt box. The user may select corresponding information by touch, gesture control, eyeball control and so on. For example, the user touches a control button on the desktop of the terminal to select "up", "down" or "enter".

Further, a wireless connection with the device associated with the smart mat may be created; and then a booting instruction and the instruction for playing the recommended content are sent to the device through the wireless connection, so as to implement automatic booting and play the recommended content.

It may be seen from the foregoing embodiments that the embodiments of the present disclosure determine identity information of a user on an smart mat, determines recommended content of the user according to the identity information of the user, and then sends an instruction for playing the recommended content to a device associated with the smart mat, so that the recommended content of the user on the smart mat can be played and the user does not need to select preferred content manually from a large amount of data, thus enabling an automatic content recommendation and bringing more use convenience for the user.

It is to be noted that the method may be also applied to an smart mat besides a terminal. The smart mat determines identity information of a user on the mat, determines recommended content of the user according to the identity information of the user, and then sends an instruction for playing the recommended content to a device associated with the smart mat. A specific implementation method is similar to the embodiments above, and will not be described repeatedly in details here.

In an alternative implementation mode, when the device is a projector, the method further includes that: a posture of the user on the smart mat is determined according to the pressure area and pre-stored correspondance relationships between pressure areas and postures, wherein the posture may include a sitting posture, a supine position and so on, a projection direction of the projector is determined according to the determined posture and pre-stored corresponding relationships between pre-stored projection directions and postures; and then the projection direction is sent to the projector associated with the smart mat, thus enabling automatically controlling a direction of the projector and improving user experience.

In an alternative implementation mode, when the identity information of the user cannot be determined, information on the top of a recent playing list may be dedtermined as the preference content to be played or recommended. Alternatively, the content played last time is determined as the preference content to be continued by default.

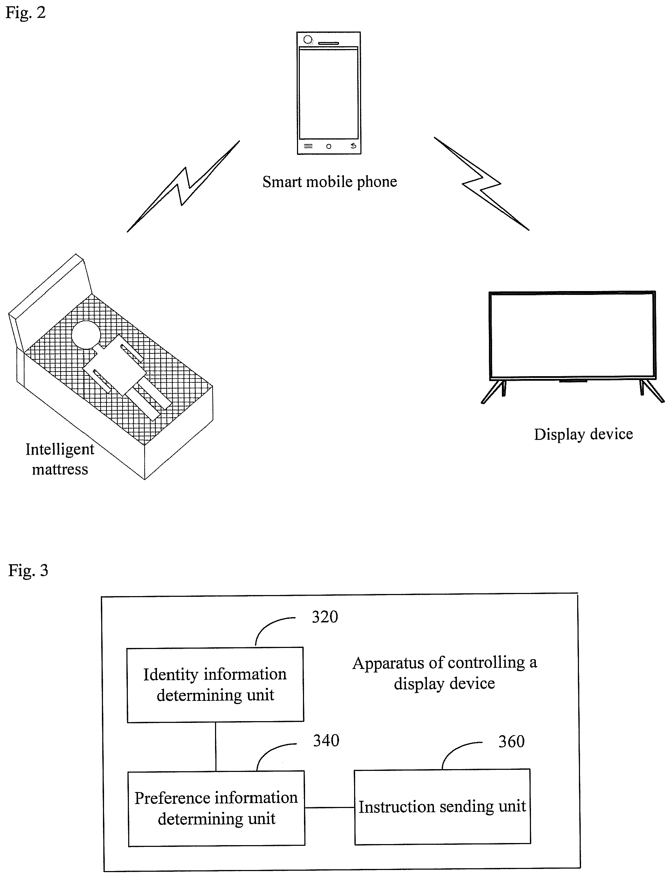

As shown in Fig. 2, Fig. 2 is a schematic diagram of an application scenario of a method for controlling a device according to an exemplary embodiment of the present disclosure. The scenario as shown in Fig. 2 includes: a smart phone functioning as a terminal, a smart mat, and a device associated with the smart mat. A user lies on the smart mattress. Several pressure sensors and temperature sensors are arranged on the smart mattress, which are at the intersections of the mesh in Fig. 2. A network communication module capable of communicating with the smart phone is provided in the smart mattress. A network communication module capable of communicating with the smart phone is also provided in the device.

The terminal controls the pressure sensors on the smart mattress to acquire pressure information, the shape of an object on the smart mat is determined according to points where the pressure information is acquired on the smart mat, the shape is compared with a pre-stored shape of human body to determine that the object lying on the mattress is a user, then the terminal reads network account information used by the user to log in an App, and pre-stored identity information of the user is read based on the network account information, and recomended content is determined for the user according to the identity information of the user, and an instruction for playing the recommended content is sent to the device associated with the smart mat. This solution thus enables the device to automatically play the recommended content for the user and improving the efficiency in acquiring the recomended content.

In the application scenario as shown in Fig. 2, a specific process for controlling the device may refer to the description of Fig. 1A, and will not be described repeatedly here.

Corresponding to the embodiments of the method for controlling the device, the embodiments of the present disclosure further provide embodiments of an apparatus for controlling a device, an apparatus and an smart mat.

As shown in Fig. 3, Fig. 3 is a block diagram of an apparatus for controlling a device according to an exemplary embodiment of the present disclosure. The apparatus includes: an identity information determining unit 320, a recomended content determining unit 340 and an instruction sending unit 360.

The identity information determining unit 320 is configured to determine identity information of a user on an smart mat;
the recomended content determining unit 340 is configured to determine recomended content for the user according to the identity information of the user; and
the instruction sending unit 360 is configured to send an instruction for playing the recomended content to a device associated with the smart mat.

It may be seen from the embodiment above that the embodiment of the present disclosure determines identity information of a user on an smart mat, determines recomended content for the user according to the identity information of the user, and then sends an instruction for playing the recomended content to a play device associated with the smart mat, so that the recomended content for the user situated on the smart mat is automatically played for the user of the mat, thus improving the content playing happening on the mat and bringing more use convenience for the user.

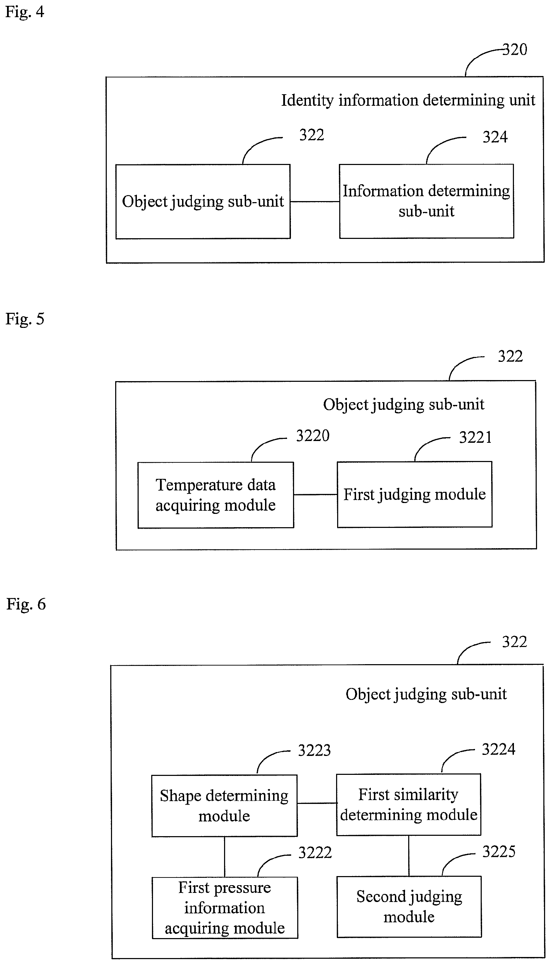

As shown in Fig. 4, Fig. 4 is a block diagram of another apparatus for controlling a device according to an exemplary embodiment of the present disclosure. The embodiment is based on the embodiment as shown in Fig. 3, the identity information determining unit 320 includes an object judging sub-unit 322 and an information determining sub-unit 324.

The object judging sub-unit 322 is configured to detect whether an object on the smart mat is a user; and
the information determining sub-unit 324 is configured to determine, when a user is detected, identity information of the user.

It may be seen from the embodiment above that the embodiment of the present disclosure first determines whether an object on the smart mat is a user, and when the object is a user, determines identity information of the user, so that other objects which are not human bodies are filtered, only a user is identified to determine identity information, thus improving the efficiency in determining identity information.

As shown in Fig. 5, Fig. 5 is a block diagram of still another apparatus for controlling a device according to an exemplary embodiment of the present disclosure. The embodiment is based on the embodiment as shown in Fig. 4, the object judging sub-unit 322 includes a temperature data acquiring module 3220 and a first judging module 3221.

The temperature data acquiring module 3220 is configured to acquire temperature data of the object on the smart mat; and
the first judging module 3221 is configured to determine that the object on the smart mat is a user when the temperature data is in a pre-stored range of human body temperature.

It may be seen from the embodiment above that, in the embodiment of the present disclosure, temperature data of the object on the smart mat is acquired and compared with a pre-stored range of human body temperature, and the object is determined to be a user only when the temperature data is in the pre-stored range of human body temperature, thereby improving the judgment efficiency.

As shown in Fig. 6, Fig. 6 is a block diagram of still another apparatus for controlling a device according to an exemplary embodiment of the present disclosure. The embodiment is based on the embodiment as shown in Fig. 4, the object judging sub-unit 322 includes a first pressure information acquiring module 3222, a shape determining module 3223, a first similarity determining module 3224 and a second judging module 3225.

The first pressure information acquiring module 3222 is configured to acquire pressure information on the smart mat;
the shape determining module 3223 is configured to determine the shape of the object on the smart mat according to points where the pressure information can be acquired on the smart mat;
the first similarity determining module 3224 is configured to compare the determined shape with a pre-stored shape of a human body, so as to obtain a first similarity; and
the second judging module 3225 is configured to determine that the object is a user when the first similarity is larger than a first preset threshold.

It may be seen from the embodiment above that, in the embodiment of the present disclosure, pressure information on the smart mat is acquired, and the shape of the object on the smart mat is determined according to points where the pressure information can be acquired on the smart mat, so that the determined shape can be compared with a pre-stored shape of a human body to judge whether the object on the smart mat is a user, thus improving the judgment efficiency.

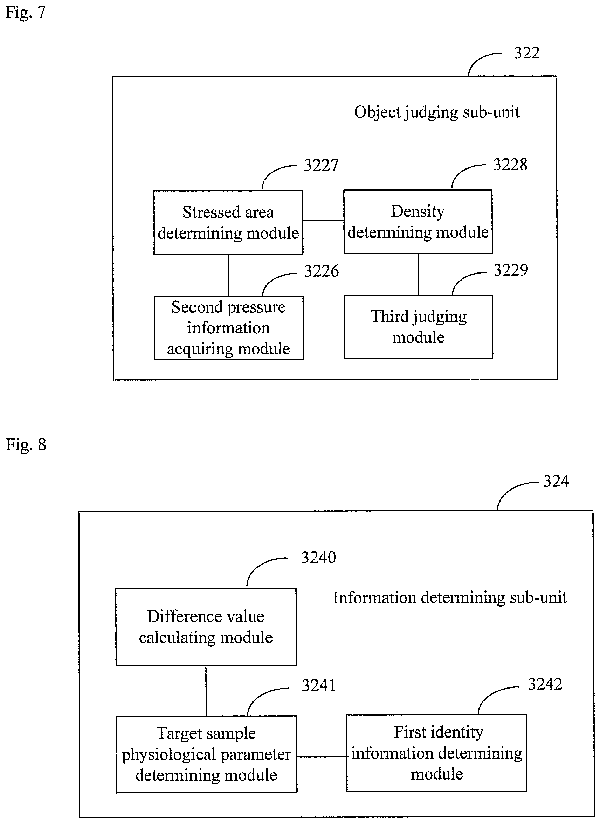

As shown in Fig. 7, Fig. 7 is a block diagram of still another apparatus for controlling a device according to an exemplary embodiment of the present disclosure. The embodiment is based on the embodiment as shown in Fig. 4, the object judging sub-unit 322 includes a second pressure information acquiring module 3226, a pressure area determining module 3227, a density determining module 3228 and a third judging module 3229.

The second pressure information acquiring module 3226 is configured to acquire pressure information on the smart mat;
the pressure area determining module 3227 is configured to determine a pressure area according to points where the pressure information is acquired on the smart mat;
the density determining module 3228 is configured to calculate the ratio of the pressure information to the pressure area to obtain the density of the object on the smart mat; and
the third judging module 3229 is configured to determine that the object is a user when the calculated density is in a pre-stored density range of human body.

It may be seen from the embodiment above that, in the embodiment of the present disclosure, pressure information on the smart mat is acquired, a pressure area is determined according to points where the pressure information can be acquired on the smart mat, the ratio of the pressure information to the pressure area is calculated so as to obtain the density of the object on the smart mat, and the object is determined to be a user when the calculated density is in a pre-stored density range of human body. Whether the object on the smart mat is a user is determined according to the density, thus improving the judgment accuracy.

As shown in Fig. 8, Fig. 8 is a block diagram of still another apparatus for controlling a device according to an exemplary embodiment of the present disclosure. The embodiment is based on the embodiment as shown in Fig. 7, the information determining sub-unit 324 includes a difference value calculating module 3240, a target sample physiological parameter determining module 3241, and a first identity information determining module 3242.

The difference value calculating module 3240 is configured to calculate difference values between physiological parameters and sample physiological parameters in a data list, wherein the physiological parameters include the pressure information, the pressure area or the density;
the target sample physiological parameter determining module 3241 is configured to select a target difference value in an allowable fluctuation range from the difference values, and determine a target sample physiological parameter corresponding to the target difference value; and
the first identity information determining module 3242 is configured to acquire identity information of a user corresponding to the target sample physiological parameter from the data list, wherein the data list records correspondance relationships between sample physiological parameters and identity information of users.

It may be seen from the embodiment above that, in the embodiment of the present disclosure, difference values between physiological parameters and sample physiological parameters in a data list are calculated, a target difference value in an allowable fluctuation range is selected from the difference values, so as to determine the identity information of the user, thus determining the identity information according to the physiological parameters. Since the physiological parameters have been determined when the user is determined, it is unnecessary to acquire other data when the identity information is determined, thus saving resources.

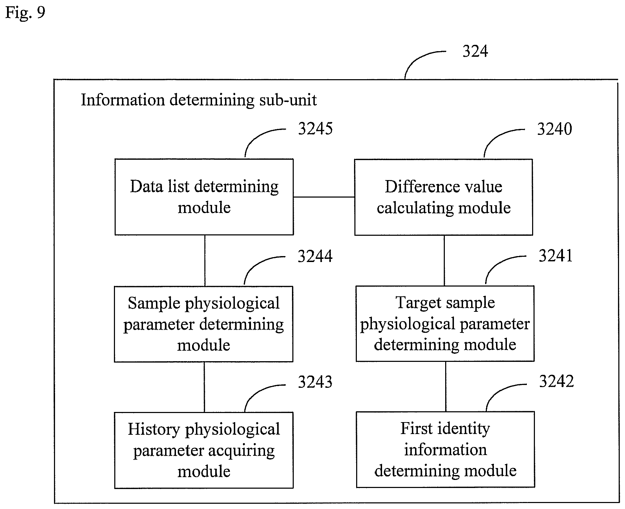

As shown in Fig. 9, Fig. 9 is a block diagram of still another apparatus for controlling a device according to an exemplary embodiment of the present disclosure. The embodiment is based on the embodiment as shown in Fig. 8, the information determining sub-unit 324 further includes a history physiological parameter acquiring module 3243, a sample physiological parameter determining module 3244 and a data list determining module 3245.

The history physiological parameter acquiring module 3243 is configured to acquire history physiological parameters of each user in a first history period from a database;
the sample physiological parameter determining module 3244 is configured to average the history physiological parameters of the each user to obtain sample physiological parameters of the each user; and
the data list determining module 3245 is configured to create correspondance relationships between identity information of users and sample physiological parameters and obtain the data list.

It may be seen from the embodiment above that, in the embodiment of the present disclosure, history physiological parameters are averaged to obtain sample physiological parameters and correspondance relationships between identity information of users and sample physiological parameters are created so as to obtain the data list, thereby improving the reliability of the sample physiological parameters.

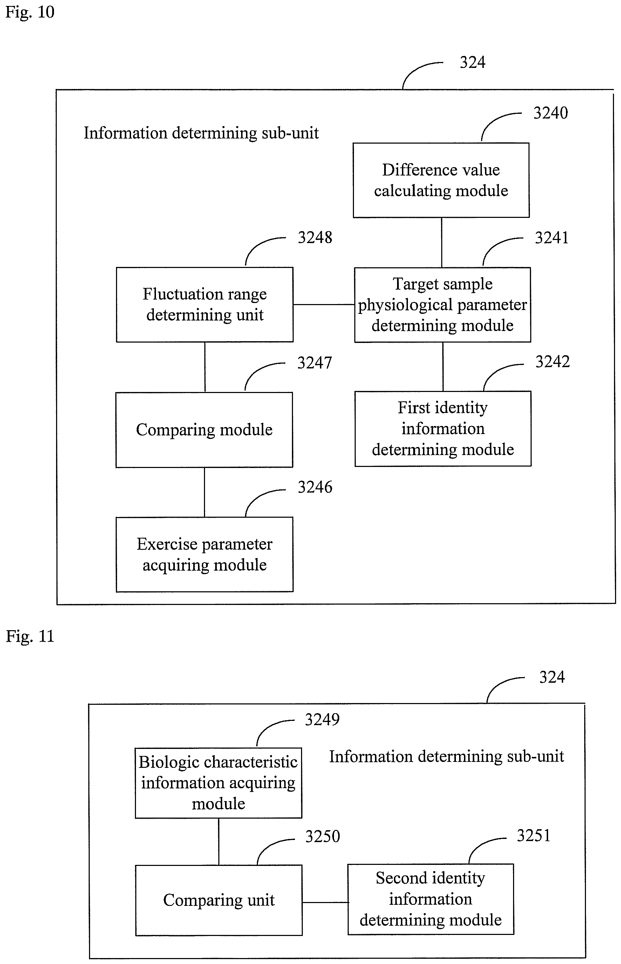

As shown in Fig. 10, Fig. 10 is a block diagram of still another apparatus for controlling a device according to an exemplary embodiment of the present disclosure. The embodiment is based on the embodiment as shown in Fig. 8, the information determining sub-unit 324 further includes an exercise parameter acquiring module 3246, a comparing module 3247 and a fluctuation range determining unit 3248.

The exercise parameter acquiring module 3246 is configured to acquire an exercise parameter of the user within a second history period;
the comparing module 3247 is configured to compare the exercise parameter with an upper exercise limit and a lower exercise limit of the user; and
the fluctuation range determining unit 3248 is configured to determine an allowable fluctuation range of the exercise parameter of the user according to a comparison result.

It may be seen from the embodiment above that, in the embodiment of the present disclosure, an exercise parameter of the user within a second history period is acquired, the exercise parameter is compared with an upper exercise limit and a lower exercise limit of the user; and an allowable fluctuation range of the exercise parameter of the user is determined according to a comparison result. The allowable fluctuation range is adjusted according to the exercise parameter, which considers a fact that the physiological parameters change with the exercise parameter, thereby improving the accuracy of the allowable fluctuation range and further improving the accuracy in determining the identity information.

As shown in Fig. 11, Fig. 11 is a block diagram of still another apparatus for controlling a device according to an exemplary embodiment of the present disclosure. The embodiment is based on the embodiment as shown in Fig. 4, the information determining sub-unit 324 includes a biologic characteristic information acquiring module 3249, a comparing unit 3250 and a second identity information determining module 3251.

The biologic characteristic information acquiring module 3249 is configured to acquire biologic characteristic information of the user, wherein the biologic characteristic information includes fingerprint information, palm print information and face information;
the comparing unit 3250 is configured to compare the acquired biologic characteristic information with pre-stored biologic characteristic information of at least one valid user; and
the second identity information determining module 3251 is configured to read, when a second similarity of the acquired biologic characteristic information to biologic characteristic information of one valid user is larger than a second preset threshold, pre-stored identity information of the one valid user.

It may be seen from the embodiment above that, in the embodiment of the present disclosure, the identity information of the user is determined by comparing acquired biologic characteristic information with pre-stored biologic characteristic information of a valid user, thereby improving the determining accuracy.

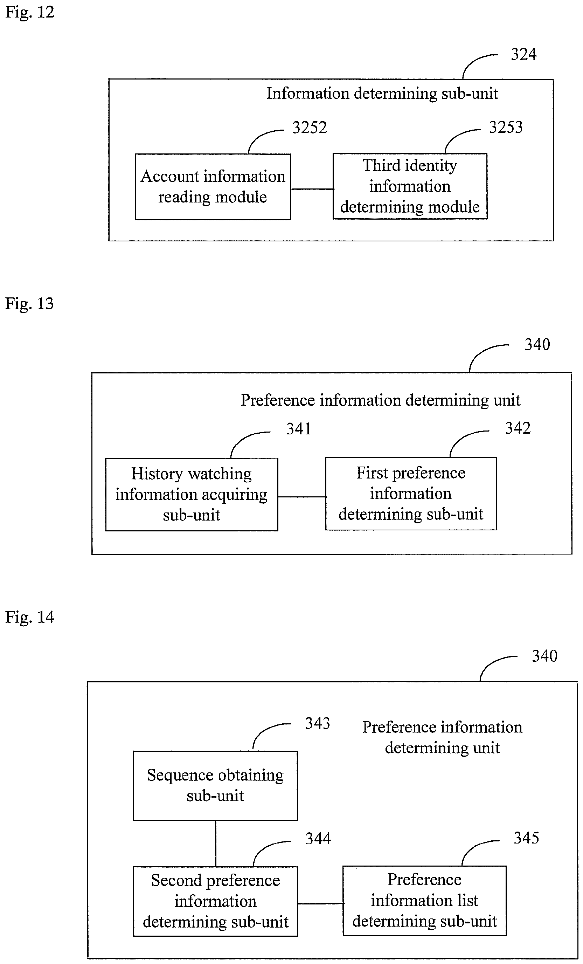

As shown in Fig. 12, Fig. 12 is a block diagram of still another apparatus for controlling a device according to an exemplary embodiment of the present disclosure. The embodiment is based on the embodiment as shown in Fig. 4, the information determining sub-unit 324 includes an account information reading module 3252 and a third identity information determining module 3253.

The account information reading module 3252 is configured to read account information used by the user to log in an App on a terminal; and
the third identity information determining module 3253 is configured to determine the identity information of the user according to the account information.

It may be seen from the embodiment above that, in the embodiment of the present disclosure, account information used by the user to log in an App on a terminal is read; and the identity information of the user is determined according to the account information, thereby improving the determining efficiency and accuracy.

As shown in Fig. 13, Fig. 13 is a block diagram of still another apparatus for controlling a device according to an exemplary embodiment of the present disclosure. The embodiment is based on the embodiment as shown in Fig. 3, the recommended content determining unit 340 includes a history playing information acquiring sub-unit 341 and a first recommended content determining sub-unit 342.

The history playing information acquiring sub-unit 341 is configured to read history playing information of the user in a third history period according to the identity information of the user; and
the first recommended content determining sub-unit 342 is configured to analyze the history playing information to acquire the recommended content for the user.

It may be seen from the embodiment above that, in the present disclosure, history playing information of the user in a third history period is read according to the identity information of the user; and the history playing information is analyzed to acquire the recommended content for the user, thereby improving the accuracy in acquiring the recommended content.

As shown in Fig. 14, Fig. 14 is a block diagram of still another apparatus for controlling a device according to an exemplary embodiment of the present disclosure. The embodiment is based on the embodiment as shown in Fig. 3, the recommended content determining unit 340 includes a sequence obtaining sub-unit 343, a second recommended content determining sub-unit 344 and a recommended content list determining sub-unit 345.

The sequence obtaining sub-unit 343 is configured to acquire, when there are at least two users on the smart mat, a sequence that the at least two users contact the smart mat;
the second recommended content determining sub-unit 344 is configured to determine recommended content corresponding to each of the at least two users according to the identity information of the at least two users; and
the recommended content list determining sub-unit 345 is configured to sort the recommended content of the at least two users according to the sequence to acquire a recommended content list.

It may be seen from the embodiment above that, in the embodiment of the present disclosure, a sequence of recommended content is determined according to a sequence that users contact the smart mat, so that a user who contacts the mat first has the priority to view his/her recommended content, thus improving user experience.

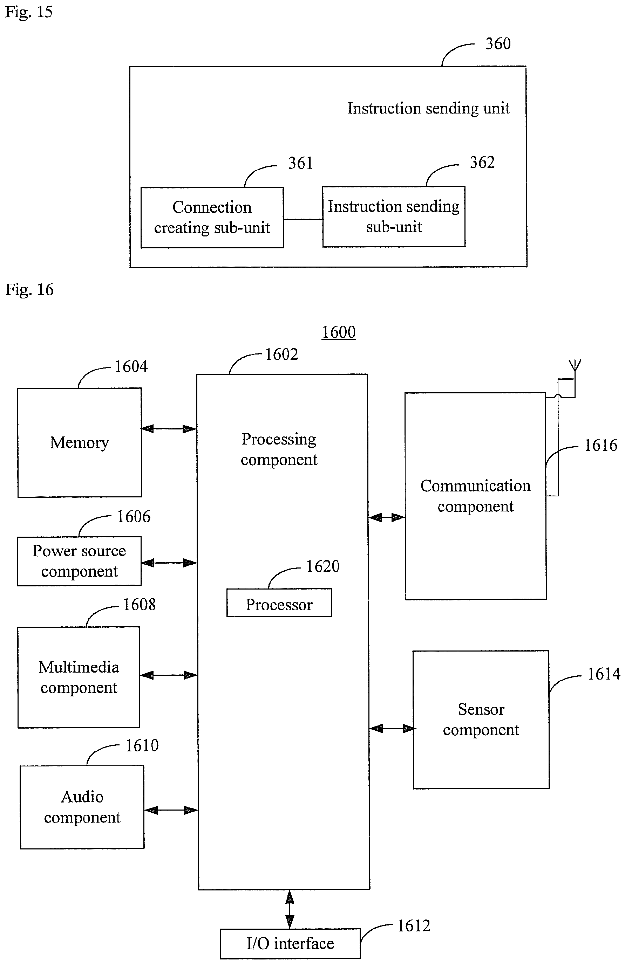

As shown in Fig. 15, Fig. 15 is a block diagram of still another apparatus for controlling a device according to an exemplary embodiment of the present disclosure. The embodiment is based on the embodiment as shown in Fig. 3, the instruction sending unit 360 includes a connection creating sub-unit 361 and an instruction sending sub-unit 362.

The connection creating sub-unit 361 is configured to create a wireless connection with the device associated with the smart mat; and

the instruction sending sub-unit 362 is configured to send a booting instruction and the instruction for playing the recommended content to the device through the wireless connection.

It may be seen from the embodiment above that, in the embodiment of the present disclosure, a booting instruction and the instruction for playing the recommended content are sent to the device so as to implement automatic booting, play recommended content and improve user experience.

Accordingly, the present disclosure further provides an apparatus, the apparatus includes a processor, and a memory configured to store an instruction executable by the processor, wherein the processor is configured to:
determine identity information of a user on an smart mat;
determine recommended content for the user according to the identity information of the user; and
send an instruction for playing the recommended content to a play device associated with the smart mat.

For details of processes for implementing functions and effect of each unit in the apparatus above, please specifically refer to processes for implementing corresponding steps in the method above, and repeated description will not be provided here, wherein the apparatus may be a terminal. For example, the apparatus may be specifically a smart phone, a tablet computer, a Personal Digital Assistant (PDA) and so on, or may be also an smart mat, that is, a chip or a device for processing information is arranged in the smart mat. The smart mat may determine identity information of a user on the smart mat, determines recommended content of the user according to the identity information of the user, and sends an instruction for playing the recommended content to a device, thus controlling the device.

Related parts of the apparatus embodiments substantially corresponding to the method embodiments may refer to the some description of the method embodiments. The apparatus embodiments described above are only schematic, wherein the units described as separate components may be or may not be physically separate. Components shown as units may be or may not be physical units, that is, may be located in one place, or may also be distributed on multiple network units. Some or all of the modules may be selected to achieve the objectives of the solutions of the embodiments of the present disclosure according to an actual requirement. A person of ordinary skills in the art can understand implement the embodiments of the present disclosure without any innovative effort.

Accordingly, the embodiments of the present disclosure further provides an smart mat, the smart mat includes:
sensors and a transmitting apparatus, wherein the sensors are uniformly arranged in a mesh pattern on the smart mat;
the sensors are configured to acquire data on the smart mat;
the transmitting apparatus is configured to transmit the data acquired by the sensors to a terminal so as to enable the terminal to determine that an object on the smart mat is a user according to the data, and control a device associated with the smart mat to play recommended content for the user.

The sensors may be pressure sensors or temperature sensors. When the sensors are pressure sensors, the pressure sensors detect pressure information on the smart mat and each pressure sensor corresponds to a detection point. When the sensors are temperature sensors, the temperature sensors detect the temperature of the object on the smart mat.

The data acquired by the smart mat is transmitted to the terminal (such as a mobile phone, a tablet computer and so on) and the terminal may run the method for controlling the device of the embodiments of the present disclosure.

As shown in Fig. 16, Fig. 16 is a structural diagram of a control apparatus 1600 applied to a device according to an exemplary embodiment of the present disclosure. For example, the apparatus 1600 may be a mobile phone, a computer, a digital broadcasting terminal, a message transceiver, a games console, a tablet device, a medical device, fitness equipment, a personal digital assistant and so on having a routing function.

Referring to Fig. 16, the apparatus 1600 may include one or more of the following components: a processing component 1602, a memory 1604, a power source component 1606, a multimedia component 1608, an audio component 1610, an Input/Output (I/O) interface 1612, a sensor component 1614 and a communication component 1616.

Generally, the processing component 1602 controls overall operations of the apparatus 1600, such as operations associated with display, a telephone call, data communication, a camera operation and a recording operation. The processing component 1602 may include one or more processors 1620 to execute instructions so as to perform all or part of the steps of the method. Besides, the processing component 1602 may include one or more modules which facilitate interaction between the processing component 1602 and other components. For example, the processing component 1602 may include a multimedia module to facilitate interaction between the multimedia component 1608 and the processing component 1602.

The memory 1604 is configured to store various types of data so as to support operations in the apparatus 1600. Examples of these data include an instruction of any App or method operated on the apparatus 1600, data of contacts, data of a telephone directory, a message, a picture, a video and so on. The memory 1604 may be implemented by a volatile or non-volatile storage apparatus of any type or a combination thereof, such as a Static Random-Access Memory (SRAM), an Electrically Erasable Programmable Read-Only Memory (EEPROM), an Erasable Programmable Read-Only Memory (EPROM), a Programmable Read-Only Memory (PROM), a Read-Only Memory (ROM), a magnetic memory, a flash memory, a magnetic disk or an optical disk.

The power source component 1606 provides power for various components of the apparatus 1600. The power source component 1606 may include a power source management system, one or more power sources, and other components associated with power generation, management and distribution of the apparatus 1600.

The multimedia component 1608 includes a screen providing an output interface between the apparatus 1600 and a user. In some embodiments, the screen may include a Liquid Crystal Display (LCD) and a Touch Panel (TP). If the screen includes a TP, the screen may be implemented as a touch screen to receive an input signal from the user. The TP includes one or more touch sensors so as to sense a touch, a slide, and a gesture on the TP. The touch sensor may not only sense a touch or a boundary of a slide, but also detect a duration and a pressure related to the touch or the slide. In some embodiments, the multimedia component 1608 includes a front camera and/or a rear camera. When the apparatus 1600 is in an operation mode, such as a camera mode or a video mode, the front camera and/or the rear camera may receive external multimedia data. Each front camera and each rear camera may be a fixed optical lens system or may be provided with a focal distance or an optical zooming capability.

The audio component 1610 is configured to output and/or input an audio signal. For example, the audio component 1610 includes a Microphone (MIC). When the apparatus 1600 is in an operation mode, such as a calling mode, a recording mode and a voice recognition mode, the MIC is configured to receive an external audio signal. The received audio signal may be further stored in the memory 1604 or sent by the communication component 1616. In some embodiments, the audio component 1610 further includes a loudspeaker, configured to output the audio signal.

The I/O interface 1612 provides an interface between the processing component 1602 and a peripheral interface module. The peripheral interface module may be a keyboard, a click wheel, a button and so on. These buttons may include, but are not limited to a homepage button, a volume button, a start button and a lock button.

The sensor component 1614 includes one or more sensors configured to provide evaluation of states of various aspects for the apparatus 1600. For example, the sensor component 1614 may detect an on/off state of the apparatus 1600, and the relative locations of the components. For example, the components are a display and a keypad of the apparatus 1600. The sensor component 1614 may further detect a change of the location of the apparatus 1600 or a change of the location of a component of the apparatus 1600, the existence or absence of contact between the user and the apparatus 1600, the orientation or acceleration/deceleration of the apparatus 1600 and a change in the temperature of the apparatus 1600. The sensor component 1614 may include a proximity sensor, configured to detect the existence of a nearby object when there is no any physical contact. The sensor component 1614 may further include an optical sensor, such as a Complementary Metal Oxide Semiconductor (CMOS) or Charge Coupled Device (CCD) image sensor used in an imaging App. In some embodiments, the sensor component 1614 may further include an acceleration sensor, a gyro sensor, a magnetic sensor, a pressure sensor or a temperature sensor.

The communication component 1616 is configured to facilitate wired or wireless communication between the apparatus 1600 and other apparatuses. The apparatus 1600 may access a wireless network based on a communication standard, such as Wireless Fidelity (WiFi), the second Generation (2G) or the third Generation (3G), or a combination thereof. In an exemplary embodiment, the communication component 1616 receives a broadcast signal from an external broadcast management system or broadcast related information through a broadcast channel. In an exemplary embodiment, the communication component 1616 further includes a Near Field Communication (NFC) module so as to promote short distance communication. For example, the NFC module may be implemented based on a Radio-frequency Identification (RFID) technology, an Infrared Data Association (IrDA) technology, an Ultra Wide Band (UWB) technology, a Bluetooth (BT) technology and other technologies.

In an exemplary embodiment, the apparatus 1600 may be implemented by one or more Application Specific Integrated Circuits (ASIC), Digital Signal Processors (DSP), Digital Signal Processing Apparatuss (DSPD), Programmable Logic Apparatuss (PLD), Field Programmable Gate Arrays (FPGA), controllers, microcontrollers, microprocessors, or other electronic components, so as to execute the method above.

A non-temporary computer readable storage medium including an instruction is further provided in an exemplary embodiment, such as the memory 1604 including an instruction. The instruction may be executed by the processor 1620 of the apparatus 1600 so as to implement the methods above. For example, the non-temporary computer readable storage medium may be a ROM, a Random-Access Memory (RAM), a Compact Disc-ROM (CD-ROM), a magnetic tape, a floppy disk, an optical data memory and so on.

A non-temporary computer readable storage medium enables a terminal to execute a method for controlling an apparatus when an instruction in the storage medium is executed by a processor of the terminal. The method includes that identity information of a user on an smart mat is determined; recommended content for the user is determined according to the identity information of the user; and an instruction for playing the recommended content is sent to a device associated with the smart mat.

## Claims

1. A method for controlling a device, comprising steps of:
determining (101), by a terminal, identity information of a user situated on an smart mat;
determining (102), by said terminal, recommended content for the user according to the identity information of the user; and
sending (103), by said terminal, an instruction for playing the recommended content to a device associated with the smart mat,
the method being **characterized in that**:the step of determining (101) identity information of a user situated on an smart mat comprises steps of:
detecting whether an object on the smart mat is a user; and
if a user is detected, determining identity information of the user,
wherein the step of determining (101) identity information of the user comprises steps of:
calculating difference values between physiological parameters and sample physiological parameters in a data list, wherein the physiological parameters comprise pressure information, pressure area or density;
selecting a target difference value within an allowable fluctuation range from the difference values, and determining a target sample physiological parameter corresponding to the target difference value; and
acquiring the identity information of the user corresponding to the target sample physiological parameter from the data list, wherein the data list records correspondence relationships between sample physiological parameters and identity information of users;
wherein a manner for creating the data list comprises steps of:
acquiring history physiological parameters of a user in a first history period from a database;
averaging the history physiological parameters of the user to obtain sample physiological parameters of the user; and
establishing correspondence relationships between identity information of the user and sample physiological parameters and creating the data list and
wherein a manner for determining the allowable fluctuation range comprises steps of:
acquiring an exercise parameter of the user from a smart wrist band within a second history period;
comparing the exercise parameter with an upper exercise limit and a lower exercise limit of the user; and
determining the allowable fluctuation range according to a comparison result of the comparison.

2. The method according to claim 1, wherein the step of detecting whether an object on the smart mat is a user comprises steps of:
acquiring temperature data of the object on the smart mat; and
if the temperature data is within a pre-stored temperature range of human body, determining that the object on the smart mat is a user.

3. The method according to claim 1 or 2, wherein the step of detecting whether an object on the smart mat is a user comprises steps of:
acquiring pressure information of the object on the smart mat;
determining a shape of the object on the smart mat according to points where the pressure information is acquired on the smart mat;
comparing the determined shape with a pre-stored shape of a human body to obtain a first similarity; and
when the first similarity is larger than a first preset threshold, determining that the object is a user.

4. The method according to any one of claims 1-3, wherein the step of detecting whether an object on the smart mat is a user comprises:
acquiring pressure information of the object on the smart mat;
determining a pressure area according to points where the pressure information is acquired on the smart mat;
according to a ratio of the pressure information to the pressure area, acquiring density of the object on the smart mat; and
if the density is within a pre-stored density range of human body, determining that the object is a user.

5. The method according to any one of claims 1-4, wherein the step of determining recommended content for the user according to the identity information of the user comprises steps of:
reading history playing information of the user during a third history period according to the identity information of the user; and
analyzing the history playing information to acquire the recommended content for the user.

6. The method according to any one of claims 1-5, wherein the step of determining recommended content for the user according to the identity information of the user comprises steps of:
when there are at least two users on the smart mat, acquiring a sequence that the at least two users contact the smart mat;
determining recommended content corresponding to each of the at least two users according to the identity information of the at least two users; and
sorting the recommended content of the at least two users according to the sequence to acquire a recommended content list.

7. The method according to any one of claims 1-6, wherein the step of sending an instruction for playing the recommended content to a device associated with the smart mat comprises steps of:
creating a wireless connection with the device associated with the smart mat; and
sending a booting instruction and the instruction for playing the recommended content to the device through the wireless connection.

8. A terminal for controlling a device, comprising:
an identity information determining unit (320), configured to determine identity information of a user situated on an smart mat;
a recommended content determining unit (340), configured to determine recommended content for the user according to the identity information of the user; and
an instruction sending unit(360), configured to send an instruction for playing the recommended content to a device associated with the smart mat, **characterized in that**:
the identity information determining unit (320) is further configured to :
detect whether an object on the smart mat is a user; and
determine identity information of the user if a user is detected,
wherein the identity information determining unit (320) is further configured to :
calculate difference values between physiological parameters and sample physiological parameters in a data list, wherein the physiological parameters comprise pressure information, pressure area or density;
select a target difference value within an allowable fluctuation range from the difference values, and determining a target sample physiological parameter corresponding to the target difference value; and
acquire the identity information of the user corresponding to the target sample physiological parameter from the data list, wherein the data list records correspondence relationships between sample physiological parameters and identity information of users;
acquire history physiological parameters of a user in a first history period from a database;
average the history physiological parameters of the user to obtain sample physiological parameters of the user; and establish correspondence relationships between identity information of the user and sample physiological parameters and creating the data list and
acquire an exercise parameter of the user from a smart wrist band within a second history period;
compare the exercise parameter with an upper exercise limit and a lower exercise limit of the user; and
determine the allowable fluctuation range according to a comparison result of the comparison.

9. A computer program including instructions for executing the steps of a method for controlling a device according to any one of claims 1 to 7 when said program is executed by a terminal.

10. A recording medium readable by a terminal and having recorded thereon a computer program including instructions for executing the steps of a method for controlling a device according to any one of claims 1 to 7.

## Patentansprüche

1. Verfahren zur Steuerung einer Vorrichtung, umfassend die Schritte:
Bestimmen (101) mittels eines Endgerätes von Identitätsinformationen eines Benutzers, der sich auf einer intelligenten Matte befindet,
Bestimmen (102) mittels des Endgerätes von empfohlenem Inhalt für den Benutzer anhand der Identitätsinformationen des Benutzers und
Senden (103) mittels des Endgerätes einer Anweisung zum Abspielen des empfohlenen Inhalts an eine Vorrichtung, die der intelligenten Matte zugeordnet ist,
wobei das Verfahren **dadurch gekennzeichnet ist, dass**: der Schritt des Bestimmens (101) von Identitätsinformationen eines Benutzers, der sich auf einer intelligenten Matte befindet, die Schritte umfasst:
Erkennen, ob ein Objekt auf der intelligenten Matte ein Benutzer ist, und
wenn ein Benutzer erkannt wird, Ermitteln von Identitätsinformationen des Benutzers,
wobei der Schritt des Ermittelns (101) von Identitätsinformationen des Benutzers die Schritte umfasst:
Berechnen von Differenzwerten zwischen physiologischen Parametern und physiologischen Probenparametern in einer Datenliste, wobei die physiologischen Parameter Druckinformationen, Druckfläche oder Dichte umfassen,
Auswählen eines Zieldifferenzwerts innerhalb eines zulässigen Schwankungsbereichs aus den Differenzwerten und Bestimmen eines physiologischen Zielprobenparameters, der dem Zieldifferenzwert entspricht, und
Erlangen der Identitätsinformation des Benutzers entsprechend dem physiologischen Zielprobenparameter aus der Datenliste, wobei die Datenliste Korrespondenzbeziehungen zwischen physiologischen Probenparametern und Identitätsinformationen von Benutzern aufzeichnet,
wobei ein Weg zum Erstellen der Datenliste die Schritte umfasst:
Erlangen von historischen physiologischen Parametern eines Benutzers in einer ersten Verlaufsperiode aus einer Datenbank,
Mitteln der historischen physiologischen Parameter des Benutzers, um physiologische Probenparameter des Benutzers zu erhalten, und
Herstellen von Korrespondenzbeziehungen zwischen Identitätsinformationen des Benutzers und physiologischen Probenparametern und Erstellen der Datenliste und
wobei ein Weg zum Bestimmen des zulässigen Schwankungsbereichs die Schritte umfasst:
Erlangen eines Übungsparameters des Benutzers von einem intelligenten Armband innerhalb einer zweiten Verlaufsperiode,
Vergleichen des Übungsparameters mit einer oberen Übungsgrenze und einer unteren Übungsgrenze des Benutzers und
Bestimmen des zulässigen Schwankungsbereichs gemäß einem Vergleichsergebnis des Vergleichs.

2. Verfahren nach Anspruch 1, wobei der Schritt des Erkennens, ob ein Objekt auf der intelligenten Matte ein Benutzer ist, die Schritte umfasst:
Erlangen von Temperaturdaten des Objekts auf der intelligenten Matte und
wenn die Temperaturdaten innerhalb eines vorab gespeicherten Temperaturbereichs des menschlichen Körpers liegen, Bestimmen, dass das Objekt auf der intelligenten Matte ein Benutzer ist.

3. Verfahren nach Anspruch 1 oder 2, wobei der Schritt des Erkennens, ob ein Objekt auf der intelligenten Matte ein Benutzer ist, die Schritte umfasst:
Erlangen von Druckinformationen des Objekts auf der intelligenten Matte,
Bestimmen einer Form des Objekts auf der intelligenten Matte gemäß Punkten, an denen Druckinformation auf der intelligenten Matte erlangt werden,
Vergleichen der bestimmten Form mit einer vorher gespeicherten Form eines menschlichen Körpers, um eine erste Ähnlichkeit zu erhalten, und
wenn die erste Ähnlichkeit größer als ein erster voreingestellter Schwellenwert ist, Bestimmen, dass das Objekt ein Benutzer ist.

4. Verfahren nach einem der Ansprüche 1 bis 3, wobei der Schritt des Erkennens, ob ein Objekt auf der intelligenten Matte ein Benutzer ist, umfasst:
Erlangen von Druckinformationen des Objekts auf der intelligenten Matte,
Bestimmen eines Druckbereichs gemäß Punkten, an denen die Druckinformationen auf der intelligenten Matte erlangt werden,
gemäß einem Verhältnis der Druckinformationen zu der Druckfläche, Erlangen der Dichte des Objekts auf der intelligenten Matte, und
wenn die Dichte innerhalb eines vorab gespeicherten Dichtebereichs des menschlichen Körpers liegt, Bestimmen, dass das Objekt ein Benutzer ist.

5. Verfahren nach einem der Ansprüche 1 bis 4, wobei der Schritt des Bestimmens des empfohlenen Inhalts für den Benutzer gemäß der Identitätsinformationen des Benutzers die Schritte umfasst:
Lesen von historischen Abspielinformationen des Benutzers während einer dritten Verlaufsperiode gemäß den Identitätsinformationen des Benutzers und
Analysieren der historischen Abspielinformationen, um den empfohlenen Inhalt für den Benutzer zu erlangen.

6. Verfahren nach einem der Ansprüche 1 bis 5, wobei der Schritt des Bestimmens des empfohlenen Inhalts für den Benutzer gemäß der Identitätsinformation des Benutzers die Schritte umfasst:
wenn mindestens zwei Benutzer auf der intelligenten Matte sind, Erlangen einer Sequenz, in der die mindestens zwei Benutzer die intelligente Matte kontaktieren,
Bestimmen eines empfohlenen Inhalts entsprechend jedem der mindestens zwei Benutzer gemäß den Identitätsinformationen der mindestens zwei Benutzer und
Sortieren des empfohlenen Inhalts der mindestens zwei Benutzer gemäß der Sequenz, um eine empfohlene Inhaltsliste zu erlangen.

7. Verfahren nach einem der Ansprüche 1 bis 6, wobei der Schritt des Sendens einer Anweisung zum Abspielen des empfohlenen Inhalts an eine Vorrichtung, die der intelligenten Matte zugeordnet ist, die Schritte umfasst:
Herstellen einer drahtlosen Verbindung mit der Vorrichtung, die der intelligenten Matte zugeordnet ist, und
Senden einer Bootanweisung und der Anweisung zum Abspielen des empfohlenen Inhalts über die drahtlose Verbindung an die Vorrichtung.

8. Endgerät zur Steuerung einer Vorrichtung, umfassend:
eine Identitätsinformations-Bestimmungseinheit (320), die konfiguriert ist, Identitätsinformation eines Benutzers zu bestimmen, der sich auf einer intelligenten Matte befindet,
eine Bestimmungseinheit (340) für empfohlenen Inhalt, die konfiguriert ist, einen empfohlenen Inhalt für den Benutzer gemäß den Identitätsinformationen des Benutzers zu bestimmen, und
eine Anweisungs-Sendeeinheit (360), die konfiguriert ist, einen Befehl zum Abspielen des empfohlenen Inhalts an eine Vorrichtung zu senden, die der intelligenten Matte zugeordnet ist, **dadurch gekennzeichnet, dass**:
die Identitätsinformations-Bestimmungseinheit (320) ferner konfiguriert ist zum:
Erkennen, ob ein Objekt auf der intelligenten Matte ein Benutzer ist, und
Bestimmen von Identitätsinformationen des Benutzers, wenn ein Benutzer erkannt wird,
wobei die Identitätsinformations-Bestimmungseinheit (320) ferner konfiguriert ist zum:
Berechnen von Differenzwerten zwischen physiologischen Parametern und physiologischen Probenparametern in einer Datenliste, wobei die physiologischen Parameter Druckinformationen, Druckfläche oder Dichte umfassen,
Auswählen eines Zieldifferenzwerts innerhalb eines zulässigen Schwankungsbereichs aus den Differenzwerten und Bestimmen eines physiologischen Zielprobenparameters, der dem Zieldifferenzwert entspricht, und
Erlangen der Identitätsinformationen des Benutzers entsprechend des physiologischen Zielprobenparameters aus der Datenliste, wobei die Datenliste Korrespondenzbeziehungen zwischen physiologischen Probenparametern und Identitätsinformationen von Benutzern aufzeichnet,
Erlangen von historischen physiologischen Parametern eines Benutzers in einer ersten Verlaufsperiode aus einer Datenbank eines Benutzers,
Mitteln der historischen physiologischen Parameter des Benutzers, um physiologische Parameter des Benutzers zu erfassen, und
Herstellen von Korrespondenzbeziehungen zwischen Identitätsinformationen des Benutzers und physiologischen Parameterparametern und Erstellen der Datenliste und
Erlangen eines Übungsparameters des Benutzers von einem intelligenten Armband innerhalb einer zweiten Verlaufsperiode,
Vergleichen des Übungsparameters mit einer oberen Übungsgrenze und einer unteren Übungsgrenze des Benutzers und
Bestimmen des zulässigen Schwankungsbereichs gemäß einem Vergleichsergebnis des Vergleichs.

9. Computerprogramm, aufweisend Anweisungen zur Ausführung der Schritte eines Verfahrens zur Steuern einer Vorrichtung nach einem der Ansprüche 1 bis 7, wenn das Programm von einem Endgerät ausgeführt wird.

10. Aufzeichnungsmedium, das von einem Endgerät gelesen werden kann und auf dem ein Computerprogramm aufgezeichnet ist, das Anweisungen zur Ausführung der Schritte eines Verfahrens zur Steuern einer Vorrichtung nach einem der Ansprüche 1 bis 7 aufweist.

## Revendications

1. Procédé de commande d'un dispositif, comprenant les étapes de :
détermination (101), par un terminal, d'informations d'identité d'un utilisateur situé sur un tapis connecté ;
détermination (102), par ledit terminal, de contenu recommandé pour l'utilisateur en fonction des informations d'identité de l'utilisateur ; et
envoi (103), par ledit terminal, d'une instruction pour lire le contenu recommandé sur un dispositif associé au tapis connecté,
le procédé étant **caractérisé en ce que** : l'étape de détermination (101) d'informations d'identité d'un utilisateur situé sur un tapis connecté comprend les étapes de :
détection du fait que l'objet sur le tapis connecté est ou non un utilisateur ; et
si un utilisateur est détecté, détermination d'informations d'identité de l'utilisateur,
dans lequel l'étape de détermination (101) d'informations d'identité de l'utilisateur comprend les étapes de :
calcul de valeurs de différence entre des paramètres physiologiques et des paramètres physiologiques d'échantillon dans une liste de données, dans lequel les paramètres physiologiques comprennent des informations de pression, une aire de pression ou une masse volumique ;
sélection d'une valeur de différence cible dans une plage de fluctuation tolérée parmi les valeurs de différence, et détermination d'un paramètre physiologique d'échantillon cible correspondant à la valeur de différence cible ; et
acquisition des informations d'identité de l'utilisateur correspondant au paramètre physiologique d'échantillon cible de la liste de données, dans lequel la liste de données enregistre des relations de correspondance entre les paramètres physiologiques d'échantillon et les informations d'identité d'utilisateurs ;
dans lequel une méthode de création de la liste de données comprend les étapes de :
acquisition de paramètres physiologiques historiques d'un utilisateur dans une première période historique à partir d'une base de données ;
calcul de moyenne des paramètres physiologiques historiques de l'utilisateur pour obtenir des paramètres physiologiques d'échantillon de l'utilisateur ; et
établissement de relations de correspondance entre les informations d'identité de l'utilisateur et les paramètres physiologiques d'échantillon et création de la liste de données et
dans lequel une méthode de détermination de la plage de fluctuation tolérée comprend les étapes de :
acquisition d'un paramètre d'effort de l'utilisateur à partir d'un bracelet au poignet connecté dans une deuxième période historique ;
comparaison du paramètre d'effort à une limite d'effort supérieure et une limite d'effort inférieure de l'utilisateur ; et
détermination de la plage de fluctuation tolérée en fonction d'un résultat de comparaison de la comparaison.

2. Procédé selon la revendication 1, dans lequel l'étape de détection du fait qu'un objet sur le tapis connecté est un utilisateur comprend les étapes de :
acquisition de données de température de l'objet sur le tapis connecté ; et
si les données de température sont dans une plage de température pré-stockée d'un corps humain, détermination que l'objet sur le tapis connecté est un utilisateur.

3. Procédé selon la revendication 1 ou 2, dans lequel l'étape de détection du fait qu'un objet sur le tapis connecté est un utilisateur comprend les étapes de :
acquisition d'informations de pression de l'objet sur le tapis connecté ;
détermination d'une forme de l'objet sur le tapis connecté en fonction de points dans lesquels les informations de pression sont acquises sur le tapis connecté ;
comparaison de la forme déterminée à une forme pré-stockée d'un corps humain pour obtenir une première similarité ; et
lorsque la première similarité est supérieure à un premier seuil prédéfini, détermination que l'objet est un utilisateur.

4. Procédé selon l'une quelconque des revendications 1 à 3, dans lequel l'étape de détection du fait qu'un objet sur le tapis connecté est ou non un utilisateur comprend :
l'acquisition d'informations de pression de l'objet sur le tapis connecté ;
la détermination d'une aire de pression en fonction de points où les informations de pression sont acquises sur le tapis connecté ;
en fonction d'un rapport des informations de pression à l'aire de pression, acquisition de la masse volumique de l'objet sur le tapis connecté ; et
si la masse volumique est dans une plage de masse volumique pré-stockée d'un corps humain, détermination que l'objet est un utilisateur.

5. Procédé selon l'une quelconque des revendications 1 à 4, dans lequel l'étape de détermination de contenu recommandé pour l'utilisateur en fonction des informations d'identité de l'utilisateur comprend les étapes de :
lecture d'informations de lecture historiques de l'utilisateur pendant une troisième période historique en fonction des informations d'identité de l'utilisateur ; et
analyse des informations de lecture historiques pour acquérir le contenu recommandé pour l'utilisateur.

6. Procédé selon l'une quelconque des revendications 1 à 5, dans lequel l'étape de détermination de contenu recommandé pour l'utilisateur en fonction des informations d'identité de l'utilisateur comprend les étapes de :
lorsqu'il y a au moins deux utilisateurs sur le tapis connecté, acquisition d'une séquence indiquant que les au moins deux utilisateurs entrent en contact avec le tapis connecté ;
détermination de contenu recommandé correspondant à chacun des au moins deux utilisateurs en fonction des informations d'identité de l'au moins deux utilisateurs ; et
tri du contenu recommandé des au moins deux utilisateurs en fonction de la séquence d'acquisition d'une liste de contenu recommandé.

7. Procédé selon l'une quelconque des revendications 1 à 6, dans lequel l'étape d'envoi d'une instruction de lecture du contenu recommandé à un dispositif associé au tapis connecté comprend les étapes de :
création d'une connexion sans fil avec le dispositif associé au tapis connecté ; et
envoi d'une instruction de démarrage et de l'instruction de lecture du contenu recommandé au dispositif par l'intermédiaire de la connexion sans fil.

8. Terminal de commande d'un dispositif, comprenant :
une unité de détermination d'informations d'identité (320), configurée pour déterminer des informations d'identité d'un utilisateur situé sur un tapis connecté ;
une unité de détermination de contenu recommandé (340), configurée pour déterminer un contenu recommandé pour l'utilisateur en fonction des informations d'identité de l'utilisateur ; et
une unité d'instruction d'envoi (360), configurée pour envoyer une instruction de lecture du contenu recommandé à un dispositif associé au tapis connecté, **caractérisé en ce que** :
l'unité de détermination d'informations d'identité (320) est en outre configurée pour :
détecter si un objet sur le tapis connecté est un utilisateur ; et
déterminer des informations d'identité de l'utilisateur si un utilisateur est détecté,
dans lequel l'unité de détermination d'informations d'identité (320) est en outre configurée pour :
calculer des valeurs de différence entre des paramètres physiologiques et des paramètres physiologiques d'échantillon dans une liste de données, dans lequel les paramètres physiologiques comprennent des informations de pression, une aire de pression ou une masse volumique ;
sélectionner une valeur de différence cible dans une plage de fluctuation tolérée parmi les valeurs de différence, et déterminer un paramètre physiologique d'échantillon cible correspondant à la valeur de différence cible ; et
acquérir les informations d'identité de l'utilisateur correspondant au paramètre physiologique d'échantillon cible de la liste de données, dans lequel la liste de données enregistre des relations de correspondance entre les paramètres physiologiques d'échantillon et les informations d'identité d'utilisateurs ;
acquérir des paramètres physiologiques historiques d'un utilisateur dans une première période historique à partir d'une base de données ;
moyenner les paramètres physiologiques historiques de l'utilisateur pour obtenir des paramètres physiologiques d'échantillon de l'utilisateur ; et
établir des relations de correspondance entre les informations d'identité de l'utilisateur et les paramètres physiologiques d'échantillon et créer la liste de données et acquérir un paramètre d'effort de l'utilisateur à partir d'un bracelet au poignet connecté dans une deuxième période historique ;
comparer le paramètre d'effort à une limite d'effort supérieure et une limite d'effort inférieure de l'utilisateur ; et
déterminer la plage de fluctuation tolérée en fonction d'un résultat de comparaison de la comparaison.

9. Programme informatique comprenant des instructions d'exécution des étapes d'un procédé de commande d'un dispositif selon l'une quelconque des revendications 1 à 7 lorsque ledit programme est exécuté par un terminal.

10. Support d'enregistrement lisible par un terminal et sur lequel est enregistré un programme informatique comprenant des instructions d'exécution des étapes d'un procédé de commande d'un dispositif selon l'une quelconque des revendications 1 à 7.
